# EUROPÄISCHE PATENTSCHRIFT

(11) **EP 1 656 112 B1**
(45) Veröffentlichungstag und Bekanntmachung des Hinweises auf die Patenterteilung: **10.02.2010**
(21) Anmeldenummer: 04729066.3
(22) Anmeldetag: 23.04.2004
(51) Int. Cl.: A61K 9/00, A61K 9/70, A61K 31/55, A61P 25/28, A61P 25/32, A61P 25/34

(54) **BUCCALE FORMULIERUNGEN DES GALANTHAMINS UND DEREN ANWENDUNGEN**
BUCCAL FORMULATIONS OF GALANTHAMINE AND USES THEREOF
FORMULES ORALES DE LA GALANTHAMINE ET LEURS APPLICATIONS

(30) Priorität: 19.08.2003 DE 10338544
(43) Veröffentlichungstag der Anmeldung: 17.05.2006
(73) Patentinhaber: Janssen Pharmaceutica NV, 2340 Beerse (BE)
(72) Erfinder: ASMUSSEN, Bodo, D-56710 Bendorf (DE); MOORMANN, Joachim, 59368 Werne (DE)
(74) Vertreter: Flaccus, Rolf-Dieter
(86) Internationale Anmeldenummer: PCT/EP2004/004325
(87) Internationale Veröffentlichungsnummer: WO 2005/027870

(56) Entgegenhaltungen:
- EP-A- 0 449 247
- WO-A-97/29750
- WO-A-02/085370
- US-A- 5 904 929

## Beschreibung

Die Erfindung betrifft filmförmige Arzneimittel zur buccalen Verabreichung von Galanthamin, dessen Salzen und Derivaten, sowie die Verwendung dieser Arzneimittel zur Behandlung von Krankheiten oder Krankheitssymptomen.

Galanthamin (4a,5,9,11,12-Hexahydro-3-methoxy-11-methyl[6H]-benzofuro-[3a,3,2ef][2]benzazepin-6-ol) ist ein gehirngängiger Inhibitor der Cholinesterase-Enzyme und ein Modulator neuronaler nicotinischer Acetylcholin-Rezeptoren (nAChR). Diese befinden sich an präsynaptischen Endigungen verschiedener Nervenbahnen, insbesondere cholinerger und dopaminerger Nervenbahnen, und können entweder durch den natürlichen Liganden Acetylcholin oder durch synthetische Liganden aktiviert werden. An nAChR wirkt Galanthamin in geringen Konzentrationen direkt als sogenannter allosterisch potenzierender Ligand (APL), der die Reaktion dieser Rezeptoren auf Acetylcholin verstärkt. Da Galanthamin in diesem Konzentrationsbereich gleichzeitig auch als Hemmstoff des katabolischen Enzyms Acteylcholinesterase die Konzentration von Acetylcholin im synaptischen Bereich erhöht, zeigt Galanthamin eine besonders ausgeprägte Verstärkung der cholinergen Neurotransmission.

Diese stark cholinerge Wirkung des Galanthamins wird zur Therapie des zentralen cholinergen Defizits bei der Alzheimer'schen Krankheit ausgenutzt. Das Hydrobromid-Salz ist in Form von direkt den Wirkstoff freisetzenden Tabletten (WO 97/47304) und einer Trinklösung jeweils unter dem Handelsnamen Reminyl zur Therapie der leichten bis mittelschweren Alzheimer'schen Demenz zugelassen.

Galanthamin und seine Salze werden für die Behandlung verschiedener weiterer Krankheiten und Krankheitssymptome verwendet, oder wurden dafür in Betracht gezogen. Zu diesen Krankheiten und Symptomen gehören:
- Lähmungszustände bei oder als Folge von: Poliomyelitis, Myasthenia gravis, Gehirn- und Rückenmarksverletzungen (Göpel et al., Psychiat. Neurol. Med. Psych. 23, 712-718(1971));
- Chronisches Müdigkeits-Syndrom (EP-B-O 584 185);
- Schizophrenie (EP-B-O 584 285);
- Schlafstörungen, insbesondere Schnarchen und Apnoe (WO 97/22339);
- Auswirkungen des Jetlags (EP-B-O 764 025);
- Störungen des Zentralnervensystems bzw. Intoxikationen, die durch Einwirkung psychotroper Substanzen verursacht wurden (DE-A-101 19 862), Vergiftungen durch Nervengifte (DE-C-43 42 174);
- Alkoholismus (DE-C-40 10 079) oder Nicotinabhängigkeit (DE-C-43 01 782; DE-A-101 34 038);
- als Antidot für die Neurolept-Analgesie (Cozanitis et al., J. Amer. Med. Assoc. 240, 108 (1978)). ,

Da Galanthamin als Hydrobromid (oder in Form eines anderen pharmakologisch akzeptablen Salzes) einerseits vollständig aus dem Gastrointestinaltrakt resorbiert wird, andererseits aber eine relativ kurze Halbwertszeit von ca. 5 h im Plasma aufweist, ergibt sich bei Verabreichung direkt (d. h. nicht verzögert) freisetzender Darreichungsformen ein sägezahnähnlicher zeitlicher Verlauf der Plasmakonzentration des Galanthamins, da zur Erzielung eines zweimal täglichen oralen Verabreichungsschemas unnötig hohe Dosen gegeben werden müssen, um die Plasmakonzentration für einen möglichst großen Teil des zwischen den Dosierungen liegenden Zeitintervalles im therapeutisch wirksamen Bereich von ca. 10 bis 25 ng/ml zu halten. Bei diesem Verabreichungsschema muß in Kauf genommen werden, daß unmittelbar nach der Verabreichung des Galanthamin-Präparates in unkontrollierter Weise Plasmakonzentrationen von deutlich über 40 ng/ml erreicht werden, was insbesondere beim vorher noch nicht mit Galanthamin therapierten Patienten zu peripheren, insbesondere gastrointestinalen und kardiovaskulären, Nebenwirkungen (Darmkrämpfe, Durchfälle, Hypotonie) führen kann.

Es wurden daher verschiedentlich Versuche unternommen, Darreichungsformen mit kontrollierter, verzögerter Freisetzung des Galanthamins zu entwickeln, die über einen Zeitraum von 24 bis 48 h einen etwa trapezförmigen zeitlichen Verlauf der Plasmakonzentration erzielen, wobei das Plateau der Konzentration für ca. 24 Stunden im therapeutisch wirksamen, aber bei den meisten Patienten noch nebenwirkungsfreien Bereich gehalten werden kann. Eine Tablette mit verzögerter Wirkstoff-Freisetzung (beschrieben in WO-A-OO 38 686) befindet sich derzeit weltweit im Zulassungsstadium für die Indikation Alzheimer'sche Demenz. Als Tabletten haben diese Darreichungsformen den Nachteil, daß sie nicht für Patienten mit Schluckbeschwerden geeignet sind, und daß zu ihrer Einnahme Flüssigkeit benötigt wird.

Andererseits wurde auch ein transdermales therapeutisches System (TTS) entwickelt, welches Galanthamin in Form seiner freien Base enthält (DE-C-43 01 783, DE-C-40 10 079), und insbesondere im Hinblick auf eine Verwendung zur Therapie des Alkoholabusus (DE-C-40 10 079) klinisch geprüft wurde. Da die für die Therapie des Alkoholverlangens therapeutisch optimalen Plasmakonzentrationen des Galanthamins den für die Demenztherapie erforderlichen ähnlich sind, könnten solche Galanthamin-enthaltende TTS auch zur Therapie der Alzheimer'schen Krankheit eingesetzt werden. Bei Formulierungen mit direkter Freisetzung werden maximale Plasmakonzentrationen des Galanthamins nach 30 bis 60 min erreicht, bei den beschriebenen Formulierungen mit verzögerter Freisetzung tritt dies nach einigen Stunden ein.

Gerade die Natur des Suchtverhaltens mit seinen immer wieder, oft nach lange anhaltender Abstinenz, auftretenden und schwer zu beherrschenden Substanzverlangen läßt jedoch auch Darreichungsformen wünschenswert erscheinen, die einen innerhalb weniger Minuten erzielbaren Eintritt der Wirkung des Galanthamins bewirken. Auch bei der Behandlung anderer Krankheiten oder Symptome kann ein schneller Wirkungseintritt erwünscht sein. Dies ist bei den beschriebenen TTS jedoch nicht der Fall.

Aufgabe der vorliegenden Erfindung war es deshalb, Darreichungsformen für die Verabreichung von Galanthamin (oder eines Salzes oder Derivates davon) zur Behandlung von Krankheiten und Krankheitssymptomen bereitzustellen, die mit einem Mangel an durch Acetylcholin vermittelter Reizleitung und/oder mit einer Dysregulation neuronaler nicotinischer Rezeptoren einhergehen oder dadurch verursacht sind, wobei einerseits ein rascher Wirkungseintritt erzielt werde soll, ohne daß dabei inakzeptable periphere Nebenwirkungen auftreten, und andererseits die erwähnten Nachteile von bekannten Darreichungsformen, insbesondere Tabletten, vermieden werden sollen.

Überraschenderweise hat sich gezeigt, daß diese Aufgaben durch filmförmige Arzneimittel zur buccalen Verabreichung nach Anspruch 1 und die davon abhängigen Ansprüche, sowie durch die Verwendung solcher Arzneimittel zur Behandlung der in den Ansprüchen 13 bis 24 genannten Krankheiten und Krankheitssymptome gelöst werden.

Nach dem vorher Gesagten wäre nämlich unbedingt zu erwarten gewesen, daß der Einsatz einer schnell freisetzenden Formulierung, bei welcher die Wirkung innerhalb weniger Minuten nach Applikation eintritt, mit erheblichen Nebenwirkungen verbunden sein müßte. Überraschenderweise stellte sich nämlich heraus, daß es möglich ist, eine buccale Darreichungsform so zu gestalten, daß der Wirkstoff innerhalb kurzer Zeit die erwünschten zentralen Wirkungen entfaltet, ohne daß dabei inakzeptable periphere Nebenwirkungen in Kauf genommen werden müssen. Dies ist gelingt dadurch, daß das Arzneimittel in Form eines filmförmigen Arzneimittels zur buccalen Verabreichung formuliert ist. Unter "buccaler Verabreichung" wird verstanden, daß das Arzneimittel den/die enthaltenen Wirkstoff(e) im Bereich der Mundhöhle freisetzt, so daß der/die Wirkstoff(e) über die Mundschleimhaut (d. h. transmucosal) resorbiert werden kann/können. Dies hat einen schnellen Wirkungseintritt zur Folge. Während der Applikationszeit nimmt der Wafer Speichelflüssigkeit auf, und der Wirkstoff gelangt durch Diffusion nach außen in die Mundhöhle und wird über die Mundschleimhaut resorbiert. Im Kontaktbereich der Applikationsfläche kann der Wirkstoff aus dem Wafer direkt an die darunter liegende Schleimhaut abgegeben werden. Die Wirkstofffreisetzung setzt schon nach einer sehr kurzen Verzögerungszeit (ca. 10 s bis 5 min) nach Beginn der Applikation ein.

Besonders geeignet sind Arzneimittel der genannten Art, welche ein leicht wasserlösliches Galanthamin-Salz (oder ein leicht wasserlösliches Salz eines Galanthamin-Derivates) in einer biokompatiblen Matrix (als Wirkstoffreservoir) enthalten, die zur Applikation in die Mundhöhle des Patienten eingebracht wird. Bevorzugt ist die genannte Matrix im Speichel löslich.

Die erfindungsgemäßen Formulierungen haben den weiteren vorteil, daß der Patient sie sich jederzeit problemlos verabreichen kann, d. h. auch dann, wenn keine Flüssigkeit verfügbar ist, oder wenn der Patient unter Schluckbeschwerden leidet. Ferner kann die Einnahme unauffälliger erfolgen als z. B. die Tabletten-Einnahme, da keine Flüssigkeit benötigt wird; dies erhöht die Bereitschaft der Patienten zur Einnahme der Arzneimittel erheblich. Die Applikation der filmförmigen Arzneimittel auf der Mundschleimhaut wird aufgrund der geringen Dicke von den behandelten Personen auch nicht als störend empfunden.
Darüber hinaus können die erfindungsgemäßen Arzneimittel für medikamentöse Therapien in der Veterinärmedizin vorteilhaft verwendet werden, besonders als mucoadhäsive Darreichungsformen.

Bei den erfindungsgemäßen filmförmigen Arzneimitteln handelt es sich um flächenförmige Darreichungsformen vorzugsweise in Form von dünnen Blättchen oder oblatenförmigen Gebilden (auch "wafer" genannt), die bevorzugt eine Gesamt-Schichtdicke im Bereich 0,01 bis 5 mm, besonders bevorzugt im Bereich von 0,03 bis 2 mm, insbesondere im Bereich von 0,05 bis 1 mm, aufweisen. Die filmförmigen Arzneimittel werden oral appliziert und sind vorzugsweise mit mucoadhäsiven Eigenschaften ausgestattet, um ein Anhaften an der Mundschleimhaut (insbesondere buccal oder sublingual, oder im Bereich des Zahnfleisches oder des Gaumens) zu ermöglichen.

Der Wafer kann als dichtes Gebilde vorliegen, wobei die Dichte vorzugsweise zwischen 0,3 g/cm³ und 1,7 g/cm³, besonders bevorzugt zwischen 0,5 g/cm³ und 1,5 g/cm³, insbesondere zwischen 0,7 g/cm³ und 1,3 g/cm³ liegt. Die Flächenform der einzelnen Wafer kann vorteilhaft rund, oval, drei- bis viereckig oder vieleckig gestaltet sein oder eine beliebige geometrische Form aufweisen.
Ferner umfaßt die Erfindung auch solche Ausführungsformen, bei denen zumindest eine Seite oder Oberfläche des Wafers, oder beide Seiten, eine Vielzahl von erhabenen Strukturen oder/und Vertiefungen aufweist, beispielsweise Noppen, Rippen oder Furchen.

Vorzugsweise enthalten die erfindungsgemäßen Arzneimittel den Wirkstoff Galanthamin in Form eines seiner wasserlöslichen, pharmazeutisch akzeptablen Salze, oder auch in Form eines Komplexsalzes, wobei Galanthamin-Hydrobromid besonders bevorzugt wird. Galanthamin kann aber auch in Form seiner freien Base in den Arzneimitteln enthalten sein. Darüber hinaus kommen als Wirkstoffe auch Galanthamin-Derivate in Betracht, die eine dem Galanthamin vergleichbare - oder auch stärkere oder schwächere - Wirkung haben, sofern sie die Blut-Hirn-Schranke passieren können und keine inakzeptablen Nebenwirkungen verursachen; ebenso die pharmazeutisch akzeptablen Salze solcher Derivate.

Geeignete Galanthamin-Derivate und Salze davon sind beispielsweise in WO-A-01 74820, EP-B-O 854 873, EP-B 0 853 624, EP-B-O 653 427, EP-B-O 648 771, EP-B-O 649 846 oder in den US-Patenten 5 958 903, 6 093 815, 6 150 354, 6 268 358, 6 319 91 beschrieben worden.
Galanthamin kann aus den Zwiebeln von Galanthus-Arten isoliert werden, beispielsweise durch das in EP-B-O 815 112 beschriebene Verfahren; alternativ kann Galanthamin auch auf synthetischem Wege hergestellt werden (z.B. Shimizu et al., Heterocycles 8, 277-282 (1977)).
Der Inhalt der vorgenannten Druckschriften gehört zur Offenbarung der vorliegenden Erfindung.

Die Erfindung umfaßt sowohl die Verwendung racemischer Gemische der genannten Wirkstoffe, als auch angereicherter oder isolierter Enantiomere.

Die erfindungsgemäßen Arzneimittel können wahlweise eine Kombination von zwei oder mehreren der vorgenannten Wirkstoffe enthalten. Der gesamte Wirkstoff-Gehalt, bezogen auf die wirkstoffhaltige(n) Schicht(en), beträgt vorzugsweise 0,1 bis 30 Gew.-%, besonders bevorzugt 1 bis 20 Gew.-%, insbesondere 5 bis 15 Gew.-%.

Die enthaltene Wirkstoffdosis liegt vorzugsweise im Bereich von 1 bis 500 mg, insbesondere 10 bis 100 mg.

Wahlweise können die erfindungsgemäßen Wafer zusätzlich mindestens einen weiteren Wirkstoff aus der Gruppe der Acetylcholinesterase-Inhibitoren enthalten, der nicht aus der Gruppe von Galanthamin und dessen Derivaten ausgewählt ist. Des weiteren können die erfindungsgemäßen Wafer zusätzlich mindestens einen Wirkstoff enthalten, der nicht aus der Gruppe der Acetylcholinesterase-Inhibitoren ausgewählt ist; beispielsweise können Wafer, die zur Behandlung des Nicotin-Abusus verwendet werden, zusätzlich Opiat-Antagonisten enthalten.

Die filmförmigen Arzneimittel weisen einen mindestens einschichtigen Aufbau auf. Die genannten Schicht, oder mindestens eine von mehreren Schichten, weist vorzugsweise eine Polymermatrix auf, die als Wirkstoffreservoir dient. Der Polymergehalt beträgt vorzugsweise 5 bis 95 %, vorzugsweise 15 bis 75 Gew.-%, besonders bevorzugt 20 bis 50 Gew.-%, bezogen auf die jeweilige Schicht.

Für die Herstellung der Polymermatrix werden insbesondere folgende Polymere bevorzugt: Cellulose-Ether, insbesondere Ethylcellulose, Hydroxyethylcellulose, Propylcellulose, Carboxymethylcellulose, Na-Carboxymethylcellulose, Hydroxypropylcellulose, Hydroxypropylmethylcellulose, Hydroxypropylethylcellulose, Gemische von Cellulose-Ethern, Cellulose-Acetat, Polyvinylalkohole (voll- oder teilhydrolysiert), Polyvinylacetat, Polyvinylpyrrolidone, Polyethylenoxid-Polymere, Polyethylenglykole, Polyurethan, Polyacrylsäure, Polyacrylate, Polymethacrylate; Poly(methylvinylether-maleinsäureanhydrid), z. B. Gantrez^{®}-Typen wie Gantrez-AN, -S, -ES, -MS, insbesondere Gantrez^{®} AN 119, Gantrez^{®} AN 117, Gantrez^{®} MS 955 (Fa. ISP); Alginate, Pectine, Gelatine; Polysaccharide, insbesondere Stärke und Stärkederivate, z. B. Tapioka-Stärke; natürliche Gummen.
Die genannten Bestandteile können auch in Kombination bzw. als Mischungen, welche zwei oder mehrere solcher Bestandteile enthalten, verwendet werden.

Nach einer bevorzugten Ausführungsform der Erfindung hat das filmförmige Arzneimittel die Eigenschaft, daß es in wässrigen Medien löslich ist und/oder darin schnell zerfällt, jedoch nicht mucoadhäsiv ist.
Unter wässrigen Medien werden insbesondere Wasser und physiologische Flüssigkeiten wie Speichel und Mucus verstanden.
Als schnell zerfallende Filme werden solche verstanden, die in einem wässrigen Medium innerhalb von 2 min, vorzugsweise 60 s, besonders bevorzugt 10 s, vollständig oder im wesentlichen vollständig zerfallen, bei einer Temperatur von 37 °C.

Nach einer weiteren bevorzugten Ausführungsform weisen die filmförmigen Arzneimittel mucoadhäsive Eigenschaften auf, um ein Festhaften an der Mundschleimhaut während der Applikationsdauer zu ermöglichen, und sind in wässrigen Medien unter den vorstehend genannten Bedingungen nicht oder nur teilweise löslich oder zerfallsfähig. "Mucuoadhäsiv" bedeutet, daß zumindest eine Seite oder Oberfläche des filmförmigen Arzneimittels mucoadhäsiv ist; "nur teilweise löslich oder zerfallsfähig" bedeutet, daß während der Dauer der Applikation (ca. 2 h bis 24 h) weniger als 50 Gew.-%, vorzugsweise weniger als 70 Gew.-%, insbesondere weniger als 90 Gew.-% der Zubereitung (ohne Berücksichtigung der freigesetzten Wirkstoffmenge) im ungelösten oder nicht zerfallenen Zustand vorhanden sind.

Nach einer weiteren bevorzugten Ausführungsform zeichnen sich die filmförmigen Zubereitungen dadurch aus, daß sie entweder mucoadhäsiv und in wässrigen Medien löslich oder zerfallsfähig sind, oder daß sie mucoadhäsiv und in wässrigen Medien gelierbar oder quellfähig sind. Vorzugsweise beträgt die Zerfallszeit 10 s bis 12 h, besonders bevorzugt 1 min bis 1 h, insbesondere 3 min bis 15 min.

Die mucoadhäsiven Eigenschaften sowie die Zerfallseigenschaften und Löslichkeitseigenschaften werden im wesentlichen durch die Art des/der die Matrix bildenden Polymers/Polymere, sowie die relativen Anteile dieser Polymere, bestimmt.

Als matrixbildende Polymere, welche Bestandteile einer erfindungsgemäßen mucoadhäsiven Formulierung sein können, kommen - ohne andere geeignete, dem Fachmann bekannte Rohstoffe auszuschließen - vorzugsweise folgende Polymere in Betracht, die einzeln oder in verschiedenen Kombinationen verwendet werden können: Polyvinylalkohole (z. B. Mowiol^{®}), Cellulose-Derivate wie Hydroxypropylmethylcellulose, Hydroxypropylcellulose, Natrium-Carboxymethylcellulose (z. B. Walocel^{®}), Methylcellulose, Hydroxyethylcellulose und Hydroxypropylethylcellulose; Stärke und Stärkederivate; Gelatine (verschiedene Typen); Polyvinylpyrrolidone; Gummi arabicum und andere Gummen; Pullulan; Acrylate; Polyacrylamid.

Als wasserlösliche (oder zerfallsfähige) oder/und quellfähige oder/und gelbildende Polymere eignen sich insbesondere Polymere aus folgender Gruppe: Stärke und Stärkederivate, Dextran; Cellulose-Derivate (wie oben beschrieben; sowie Carboxymethylcellulose, Ethylcellulose, Propylcellulose); Polyvinylalkohole, Polyvinylacetat, Polyacrylsäure, Polyacrylate, Polyvinylpyrrolidone, Polyethylenoxid-Polymere, Polyacrylamide, Polyethylenglykol, Gelatine, Kollagen und andere gelbildende Proteine; Alginate, Pectine, Pullulan, Xanthan, Traganth, Chitosan, Alginsäure, Arabinogalactan, Galactomannan, Agar-Agar, Agarose, Carrageenan, natürliche Gummen. Die genannten Stoffe können einzeln oder in verschiedenen Kombinationen verwendet werden.

Die erfindungsgemäßen filmförmigen Arzneimittel können ferner auch in Form von verfestigten Schäumen hergestellt werden. Diese Ausführungsform wird insbesondere bei in wässrigen Medien schnell zerfallenden filmförmigen Zubereitungen bevorzugt. Aufgrund ihrer großen inneren Oberfläche und der relativ steifen Ausführung zeichnen sie sich einerseits durch ein hervorragendes "mouthfeel" aus, und andererseits ermöglichen sie eine besonders schnelle Wirkstofffreisetzung. Die Dichte dieser trockenen Schäume liegt bevorzugt zwischen 0,01 g/cm³ und 0,9 g/cm³, besonders bevorzugt zwischen 0,08 g/cm³ und 0,4 g/cm³, insbesondere zwischen 0,1 g/cm³ und 0,3 g/cm³. Das für die Berechnung der Dichte verwendete Volumen wird dabei durch das den Gesamtkörper des Wafers ausgefüllte Volumen definiert. Derartige schaumförmige Wafer können beispielsweise durch das in DE-A-100 32 456 beschriebene Verfahren hergestellt werden.

Die Herstellung der erfindungsgemäßen filmförmigen.Arzneimittel erfolgt im allgemeinen in der Weise, daß zunächst eine Beschichtungsmasse hergestellt wird, welche Matrixpolymer(e), Wirkstoff(e) und gegebenenfalls Hilfsstoffe in einem Lösungsmittel oder -gemisch enthält, und diese Masse auf einer inerten Unterlage zu einem feuchten Film ausgestrichen wird, z. B. durch Rakel-, Walzenauftrags-, Sprühoder Extrusionsverfahren. Dieser Film wird getrocknet (oder erstarren gelassen) und nach Bedarf in Dosiseinheiten gewünschter Flächengröße (und mit definiertem Wirkstoffgehalt) zerteilt. Bevorzugt wird der getrocknete Film in Flächenabschnitte zerteilt, deren Größe weniger als 10 cm², besonders bevorzugt weniger als 8 cm², und insbesondere weniger als 4 cm² beträgt. Auch eine Herstellung aus einer Schmelze, welche die genannten Bestandteile enthält, kommt in Betracht.

Gemäß einer bevorzugten Ausführungsform zeichnen sich die erfindungsgemäßen Wafer dadurch aus, daß sie innerhalb von 30 min, vorzugsweise innerhalb von 15 min, besonders bevorzugt innerhalb von 5 min, nach der Applikation den/die enthaltenen Wirkstoff(e) in der Mundhöhle freisetzen, so daß ein wirksamer Plasmaspiegel erreicht wird.

Ferner ist vorgesehen, daß das filmförmige Arzneimittel wahlweise zwei- oder mehrschichtig aufgebaut ist, wobei mindestens eine der Schichten wirkstoffhaltig ist. Die einzelnen Schichten können sich hinsichtlich eines oder mehrerer der folgenden Parameter unterscheiden: Polymerzusammensetzung, Art der Wirkstoffe, Wirkstoffkonzentration, Löslichkeits- oder Zerfallseigenschaften, Quellfähigkeit, mucoadhäsive Eigenschaften, Gehalt an Hilfsstoffen. Mehrschichtige Filme können beispielsweise in der Weise erhalten werden, daß zunächst eine erste Filmschicht hergestellt wird (wie beschrieben) und auf diese nach dem Trocknen eine weitere Schicht aufgetragen wird. Alternativ können zwei oder mehrere Schichten in separaten Verfahrensschritten hergestellt und diese aufeinander laminiert werden.

Gemäß einer besonders bevorzugten Ausführungsform weist die wirkstoffhaltige Schicht der Wafer, oder zumindest eine der Schichten, einen verzögerten Zeitverlauf der Wirkstofffreisetzung auf. Dadurch wird eine Wirkstoff-Freisetzung über einen Zeitraum von vorzugsweise bis zu 6 h, besonders bevorzugt bis zu 12 h, und am meisten bevorzugt bis zu 24 h ermöglicht. Die Verzögerung der Freisetzung kann durch dem Fachmann bekannte Maßnahmen erzielt werden, beispielsweise durch die Zusammensetzung (Polymere, Hilfstoffe), Dichte und Wasserunlöslichkeit der jeweiligen Matrixschicht, durch eine Steuermembran oder durch Einkapselung des Wirkstoffs in Polymerpartikel. Der Zeitverlauf der Wirkstofffreisetzung läßt sich mit den beschriebenen Maßnahmen auf vielfältige Weise steuern.

Im Falle von mehrschichtigen filmförmigen Zubereitungen wird bevorzugt, daß diese eine mucoadhäsive Schicht aufweisen, die vorzugsweise wasserlöslich oder zerfallsfähig ist, und den/die Wirkstoff(e) enthält. Auf diese Schicht folgt in distaler Richtung (d. h. zur Mundhöhle hin) mindestens eine weitere Schicht, die vorzugsweise eine verzögerte Wirkstofffreisetzung aufweist/aufweisen. Auf diese Weise wird einerseits ein rascher Wirkungseintritt und andererseits die Abgabe einer Erhaltungsdosis über einen längeren Zeitraum ermöglicht.

Des weiteren kann von der Maßnahme Gebrauch gemacht werden, daß mindestens eine der distalen Schichten als eine in wässrigen Medien lösliche oder zerfallsfähige Schicht ausgebildet ist, um eine schnelle initiale Wirkstofffreisetzung zu gewährleisten. Ferner kann wahlweise eine der Schichten, vorzugsweise eine der distalen Schichten, insbesondere die äußerste Schicht, als Sperrschicht ausgebildet sein, um die Diffusion von Wasser und/oder Wirkstoff zu verlangsamen oder zu verhindern. Diese Sperrschicht ist in wässrigen Medien nicht oder nur langsam löslich und vorzugsweise wirkstofffrei.

Die erfindungsgemäßen Formulierungen können zusätzlich einen oder mehrere Hilfsstoffe enthalten, insbesondere aus folgenden Gruppen:
Füllstoffe (z. B. SiO₂, Titandioxid, Zinkoxid, Kreide, Aktivkohle, Maisstärke); Farbstoffe;
Emulgatoren (z. B. polyethoxylierte Sorbitanfettsäureester, polyethoxylierte Fettalkohole, Lecithin);
Weichmacher (z. B. Polyethylenglykol, Glycerin, Sorbitol, Mannitol und andere Zuckeralkohole, Dexpanthenol; Polyalkohole wie Glycerin, Propandiol, Butandiol, Mygliol; höhere Alkohole wie Dodecanol, Undecanol, Octanol; Triglyceride), Zerfallsförderer, Sprengmittel (Dochtmittel, z. B. Aerosil);
Netzmittel; Süß- und Aromastoffe (z. B. Pfefferminze, Minze, Menthol, Campher); Antioxidantien;
Konservierungsmittel (z. B. Sorbinsäure und deren Salze, Vitamine A und E); pH-Regulatoren;
Permeations- oder resorptionsfördernde Substanzen (z. B. gesättigte oder ungesättigte Fettsäuren; Fettsäure-Ester, insbesondere Ester mit Methanol, Ethanol und Isopropanol, z. B. Ölsäureethylester, Ölsäuremethylester, Laurinsäureethylester, Laurinsäuremethylester, Adipinsäuremethylester, Adipinsäureethylester; Fettalkohole und deren Ester, insbesondere Ester mit Essig- oder Milchsäure, z. B. Ethyloleat, Ethyllaurat, Ethylpalmitat; mehrwertige aliphatische Alkohole wie Propandiol, oder Polyethylenglykole; Sorbitanfettsäureester und deren durch Ethoxylierung erhältlichen Derivate; Fettalkoholethoxylate, Polyoxyethylenfettsäureester, Laurinsäurediethanolamid, Ölsäurediethanolamid; Tocopherole; Laurinsäurehexylester; 2-Octyldodecanol, Dexpanthenol, Isopropylidenglycerol, Transcutol, DEET, Solketal; Menthol und andere ätherische Öle oder Bestandteile solcher Öle; sowie Kombinationen davon).

Die genannten Hilfsstoffe können vorzugsweise in einer Gesamtkonzentration von bis zu 50 Gew.-%, insbesondere in einer Gesamtkonzentration von 1 bis 15 Gew.-% enthalten sein, jeweils bezogen auf die wirkstoffhaltige(n) Schicht(en). Durch Veränderung von Art und Menge der zugesetzten Hilfsstoffe können die chemischen oder physikalischen Eigenschaften der Wafer beeinflußt werden, wie z. B. Flexibilität, mucoadhäsive Eigenschaften, Zerfallsfähigkeit, Quellfähigkeit, Diffusionseigenschaften.

Die Erfindung erstreckt sich ferner auf die Verwendung mindestens eines zentral wirksamen cholinergen Wirkstoffes, ausgewählt aus der Galanthamin, pharmazeutisch akzeptable Salze des Galanthamins, Galanthamin-Derivate und deren pharmazeutisch akzeptablen Salze umfassenden Gruppe, zur Herstellung von filmförmigen buccalen Arzneimitteln zur transmucosalen Verabreichung des/der genannten Wirkstoffe(s) zur Behandlung von Krankheiten oder Krankheitssymptomen, die mit einem Mangel an durch Acetylcholin vermittelter Reizleitung und/oder mit einer Dysregulation neuronaler nicotinischer Rezeptoren einhergehen oder dadurch verursacht sind.
Des weiteren umfaßt die vorliegende Erfindung die Verwendung von filmförmigen buccalen Arzneimitteln mit mindestens einen zentral wirksamen cholinergen Wirkstoff, ausgewählt aus der Galanthamin, pharmazeutisch akzeptable Salze des Galanthamins, Galanthamin-Derivate und deren pharmazeutisch akzeptablen Salze umfassenden Gruppe, zur transmucosalen Verabreichung des/der genannten Wirkstoffe(s) zur Behandlung von Krankheiten oder Krankheitssymptomen, die mit einem Mangel an durch Acetylcholin vermittelter Reizleitung und/oder mit einer Dysregulation neuronaler nicotinischer Rezeptoren einhergehen oder dadurch verursacht sind.

Insbesondere eignen sich die erfindungsgemäßen filmförmigen Zubereitung für die medikamentöse Therapie folgender Krankheiten und Symptome:
Alzheimer'sche Krankheit (in allen Erscheinungsformen und Stadien), insbesondere die damit einhergehenden Gedächtnisstörungen (Alzheimer-Demenz); ferner Down-Syndrom, Spätstadien des Downsyndroms (insbesondere Demenz, Verlust kognitiver Fähigkeiten); Gedächtnisstörungen mit anderen Ursachen.
Neurologische Erkrankungen oder Symptome, insbesondere Lähmungszustände bei oder als Folge von: Poliomyelitis, Myasthenia gravis, Gehirn- und Rückenmarksverletzungen, Multipler Sklerose, Amyotrophische Lateralsklerose, Schlaganfall, Schädel-Hirn-Trauma, Tumorerkrankungen.
Chronisches Müdigkeits-Syndrom; Schizophrenie; Manie; Schlafstörungen, insbesondere Schnarchen und Apnoe; Auswirkungen des Jetlags sowie andere Störungen des physiologischen Rhythmus von Körperfunktionen;
Störungen des Zentralnervensystems, die durch Einwirkung psychotroper Substanzen verursacht wurden, insbesondere Intoxikationen mit solchen Substanzen; Vergiftungen durch Nervengifte oder Kampfstoffe (insbesondere phosphororganische Stoffe);
Störungen des Zentralnervensystems, die infolge der Einwirkung von psychotropen Substanzen als Folge von gelegentlichem oder chronischen Gebrauch oder Mißbrauch von Suchtmitteln, Rauschmitteln oder Medikamenten, oder als Nebenwirkung bei bestinunungsgemäßer, insbesondere bei wiederholter oder länger andauernder Anwendung von Arzneimitteln, oder infolge einer akuten Vergiftung, oder infolge chronischer Einwirkung von Giften auftreten; insbesondere Gedächtnisstörungen, sowie Beeinträchtigung der Gedächtnisleistung, Beeinträchtigung der Wahrnehmung, Beeinträchtigung der Bewegungskoordination;

Alkoholismus oder Nicotinabhängigkeit, Mißbrauchs anderer chemischer Substanzen; insbesondere eine Behandlung zur Reduktion des Alkoholverlangens oder zur Reduktion des Nicotinverlangens. Für diese Behandlungszwecke kann Galanthamin (oder ein Derivat davon), vorzugsweise in einer schnell freisetzenden filmförmigen buccalen Darreichungsform, auch in Kombination mit anderen, den jeweiligen Abusus bekämpfenden therapeutischen Wirkstoffe in ihren jeweils geeigneten Darreichungsformen verabreicht werden, beispielsweise in Kombination mit Opiat-Antagonisten (wie in DE-A-101 34 038) zur Behandlung des Nicotin-Abusus oder in Kombination mit antiexzitatorisch wirkenden Substanzen zur Behandlung des Alkohol-Abusus (wie in DE-A-101 29 265). Die genannten weiteren Wirkstoffe können auch in Kombination mit. Galanthamin (oder einem Derivat davon) in einem erfindungsgemäßen Wafer enthalten sein.

Die erfindungsgemäßen Wafer können ferner zur Antidot-Behandlung bei einer Neurolept-Analgesie verwendet werden. Ferner können die erfindungsgemäßen Wafer auch für weitere, hier nicht ausdrücklich genannte therapeutische Behandlungszwecke verwendet werden.

Die Erfindung umfaßt des weiteren Methoden zur Behandlung von Personen, die an einer der genannten Krankheiten leiden oder eines der genannten Symptome aufweisen oder die aus anderen Gründen einer Behandlung mit einem zentral wirksamen cholinergen Wirkstoff bedürfen. Dabei wird der zu behandelnden Person eine therapeutisch wirksame Dosis mindestens eines zentral wirksamen cholinergen Wirkstoffes aus der Galanthamin, pharmazeutisch akzeptable Salze des Galanthamins, Galanthamin-Derivate und deren pharmazeutisch akzeptable Salze umfassenden Gruppe in Form eines filmförmigen Arzneimittels buccal verabreicht, wie oben beschrieben.
Die Verabreichung erfolgt durch Einbringen der filmförmigen Zubereitung in den Mundraum (buccal, sublingual) und, im Falle von mucoadhäsiven Filmen, durch Aufkleben auf die buccale oder gingivale Schleimhaut oder auf andere Bereiche der Mundschleimhaut (z. B. Gaumen oder sublingual).

Abhängig vom Wirkstoffgehalt, der Freisetzungsrate, den Zerfallseigenschaften und der individuell benötigten Dosis wird die Applikation in Intervallen von vorzugsweise 2 bis 24 h, insbesondere 6 bis 12 h, wiederholt.
Die verabreichte Tagesdosis an Galanthamin (und/oder Galanthamin-Derivate(n)) beträgt, abhängig vom Körpergewicht der Person und anderen Faktoren, zwischen 10 und 750 mg, vorzugsweise 50 bis 500 mg.

## Patentansprüche

1. Filmförmiges Arzneimittel zur buccalen Verabreichung von Galanthamin oder eines Salzes oder Derivates davon, wobei das Arzneimittel mindestens eine Schicht aufweist, die einen zentral wirksamen cholinergen Wirkstoff, oder eine Kombination mindestens zweier solcher Wirkstoffe enthält, wobei der/die Wirkstoff(e) aus der Galanthamin, pharmazeutisch akzeptable Salze des Galanthamins, Galanthamin-Derivate und deren pharmazeutisch akzeptablen Salze umfassenden Gruppe ausgewählt ist/sind, und wobei das filmförmige Arzneimittel in wässrigen Medien löslich ist oder/und in wässrigen Medien bei einer Temperatur von 37°C innerhalb von 2 min vollständig oder im wesentlichen vollständig zerfällt, jedoch nicht mucoadhäsiv ist.

2. Filmförmiges Arzneimittel nach Anspruch 1, **dadurch gekennzeichnet, daß** das filmförmige Arzneimittel in wäßrigen Medien bei einer Temperatur von 37°C innerhalb von 60 s, vorzugsweise innerhalb von 10 s, vollständig oder im wesentlichen vollständig zerfällt.

3. Filmförmiges Arzneimittel nach Anspruch 1 oder 2, **dadurch gekennzeichnet, daß** die genannte Schicht oder zumindest eine der Schichten eine Polymermatrix aufweist, die als wirkstoffreservoir dient, wobei der Polymeranteil 5 bis 95 %, vorzugsweise 15 bis 75 Gew.-%, besonders bevorzugt 20 bis 50 Gew.-% beträgt.

4. Filmförmiges Arzneimittel nach einem der vorangehenden Ansprüche, **dadurch gekennzeichnet, daß** es innerhalb von 30 min, vorzugsweise innerhalb von 15 min, besonders bevorzugt innerhalb von 5 min nach der Applikation eine solche Menge des/der enthaltenen Wirkstoff(e) in der Mundhöhle freisetzt, so daß ein wirksamer Plasmaspiegel erreicht wird.

5. Filmförmiges Arzneimittel nach einem der vorangehenden Ansprüche, **dadurch gekennzeichnet, daß** es zwei- oder mehrschichtig aufgebaut ist, wobei mindestens eine Schicht wirkstoffhaltig ist.

6. Filmförmiges Arzneimittel nach einem der vorangehenden Ansprüche, **dadurch gekennzeichnet, daß** mindestens eine der Schichten eine verzögerte Wirkstofffreisetzung aufweist.

7. Filmförmiges Arzneimittel nach einem der vorangehenden Ansprüche, **dadurch gekennzeichnet, daß** der Wirkstoffgehalt 0,1 bis 30 Gew.-% beträgt, vorzugsweise 1 bis 20 Gew.-%, jeweils bezogen auf die wirkstoffhaltige(n) Schicht(en).

8. Filmförmiges Arzneimittel nach einem der vorangehenden Ansprüche, **dadurch gekennzeichnet, daß** das Arzneimittel Galanthamin, oder ein Salz oder Derivat des Galanthamins, in Kombination mit mindestens einem weiteren pharmazeutischen Wirkstoff enthält, vorzugsweise ausgewählt aus der Gruppe der Acetylcholinesterase-Inhibitoren.

9. Filmförmiges Arzneimittel nach einem der vorangehenden Ansprüche, **dadurch gekennzeichnet, daß** dessen Schichtdicke 0,01 bis 5 mm, vorzugsweise 0,03 bis 2 mm, besonders bevorzugt 0,05 bis 1 mm beträgt.

10. Filmförmiges Arzneimittel nach einem der vorangehenden Ansprüche, **dadurch gekennzeichnet, daß** es einen oder mehrere Hilfsstoffe enthält, ausgewählt aus der Füllstoffe, Farbstoffe, Emulgatoren, Weichmacher, Zerfallsförderer, Sprengmittel (Dochtmittel), Netzmittel, Süß- und Aromastoffe, Konservierungsmittel, pH-Regulatoren, permeationsfördernde Substanzen und Antioxidantien umfassenden Gruppe.

11. Verwendung mindestens eines zentral wirksamen cholinergen wirkstoffes, ausgewählt aus der Galanthamin, pharmazeutisch akzeptable Salze des Galanthamins, Galanthamin-Derivate und deren pharmazeutisch akzeptablen Salze umfassenden Gruppe, zur Herstellung eines filmförmigen buccalen Arzneimittels zur transmucosalen Verabreichung des/der genannten Wirkstoffe(s), das in wäßrigen Medien löslich ist oder/und in wäßrigen Medien bei einer Temperatur von 37°C innerhalb von 2 min vollständig oder im wesentlichen vollständig zerfällt, jedoch nicht mucoadhäsiv ist, zur Behandlung von Krankheiten oder Krankheitssymptomen, die mit einem Mangel an durch Acetylcholin vermittelter Reizleitung und/oder mit einer Dysregulation neuronaler nicotinischer Rezeptoren einhergehen oder **dadurch** verursacht sind.

12. Verwendung nach Anspruch 11, **dadurch gekennzeichnet, daß** das filmförmige Arzneimittel in wäßrigen Medien bei einer Temperatur von 37°C innerhalb von 60 s, vorzugsweise innerhalb von 10 s, vollständig oder im wesentlichen vollständig zerfällt.

13. Verwendung nach Anspruch 11, **dadurch gekennzeichnet, daß** es sich bei der genannten Krankheit um die Alzheimer'sche Krankheit handelt, oder daß es sich bei den genannten Symptomen um die im Verlauf dieser Krankheit auftretenden Gedächtnisstörungen handelt.

14. Verwendung nach Anspruch 11, **dadurch gekennzeichnet, daß** das es sich bei der genannten Behandlung um die Therapie des Alkohol-Abusus, insbesondere um eine Behandlung zur Reduktion des Alkoholverlangens, oder um die Therapie des Nicotin-Abusus, insbesondere um eine Behandlung zur Reduktion des Nicotinverlangens, handelt.

15. Verwendung nach Anspruch 11, **dadurch gekennzeichnet, daß** es sich bei der genannten Behandlung um eine Antidot-Behandlung nach Neurolept-Anästhesie handelt.

16. Verwendung nach Anspruch 11, **dadurch gekennzeichnet, daß** es sich bei der genannten Behandlung um eine Therapie des Mißbrauchs chemischer Substanzen oder der Abhängigkeit von solchen Substanzen, insbesondere eine Therapie der Intoxikation mit psychotropen Substanzen, handelt.

17. Verwendung nach Anspruch 11, **dadurch gekennzeichnet, daß** es sich bei den genannten Symptomen oder Krankheiten um Symptome des Jetlags oder um andere Störungen des physiologischen Rhythmus von Körperfunktionen handelt.

18. Verwendung nach Anspruch 11, **dadurch gekennzeichnet, daß** es sich bei den genannten Symptomen oder Krankheiten um das chronische Müdigkeitssyndrom oder um Schlafstörungen handelt.

19. Verwendung nach Anspruch 11, **dadurch gekennzeichnet, daß** es sich bei der genannten Krankheit um Schizophrenie oder um eine Manie handelt.

20. Verwendung nach Anspruch 11, **dadurch gekennzeichnet, daß** es sich bei den genannten Krankheiten oder Symptomen neurologische Erkrankungen und Symptome handelt, insbesondere Lähmungserscheinungen.

21. Verwendung nach Anspruch 11, **dadurch gekennzeichnet, daß** das Arzneimittel zur Behandlung von Störungen des Zentralnervensystems, die infolge der Einwirkung von psychotropen Substanzen als Folge von gelegentlichem oder chronischen Gebrauch oder Mißbrauch von Suchtmitteln, Rauschmitteln oder Medikamenten, oder als Nebenwirkung bei bestimmungsgemäßer, insbesondere bei wiederholter oder länger andauernder Anwendung von Arzneimitteln, oder infolge einer akuten Vergiftung, oder infolge chronischer Einwirkung von Giften, beim Menschen oder anderen Wirbeltieren auftreten.

22. Verwendung nach Anspruch 21, **dadurch gekenntzeichnet, daß** es sich bei den genannten Symptomen um Symptome aus der kognitive Störungen, insbesondere Gedächtnisstörungen, sowie Beeinträchtigung der Gedächtnisleistung, Beeinträchtigung der Wahrnehmung, Beeinträchtigung der Bewegungskoordination umfassenden Gruppe handelt.

## Claims

1. Film-shaped medicament for buccal administration of galanthamine or a salt or derivative thereof, said medicament comprising at least one layer which contains a cholinergic active substance acting on the central nervous system or a combination of at least two such active substances, said active substance(s) being selected from the group comprising galanthamine, pharmaceutically acceptable salts of galanthamine, galanthamine derivatives and their pharmaceutically acceptable salts, and said film-shaped medicament being soluble in aqueous media or/and disintegrating completely, or essentially completely, within 2 minutes in aqueous media at a temperature of 37°C, but not being mucoadhesive.

2. Film-shaped medicament according to claim 1, **characterized in that** said film-shaped medicament disintegrates completely, or essentially completely, in aqueous media at a temperature of 37°C within 60 s, preferably within 10 s.

3. Film-shaped medicament according to claim 1 or 2, **characterized in that** the said layer or at least one of the layers has a polymer matrix serving as active substance reservoir, the polymer content amounting to 5 to 95%, preferably 15 to 75%-wt, with particular preference 20 to 50%-wt.

4. Film-shaped medicament according to any one of the preceding claims, **characterized in that** within 30 min, preferably within 15 min, with particular preference within 5 min after application it releases such an amount of the active substance(s) contained therein to the oral cavity that an effective plasma level is achieved.

5. Film-shaped medicament according to any one of the preceding claims, **characterized in that** it is of a bilayer or multilayer structure, with at least one layer containing active substance.

6. Film-shaped medicament according to any one of the preceding claims, **characterized in that** at least one of the layers has a retarded active substance release.

7. Film-shaped medicament according to any one of the preceding claims, **characterized in that** the active substance content is 0.1 to 30%-wt, preferably 1 to 20%-wt., each relative to the active substance-containing layer(s).

8. Film-shaped medicament according to any one of the preceding claims, **characterized in that** the medicament contains galanthamine, or a salt or derivative of galanthamine, in combination with at least one further pharmaceutically active substance, preferably selected from the group of the acetylcholinesterase inhibitors.

9. Film-shaped medicament according to any one of the preceding claims, **characterized in that** its layer thickness is 0.01 to 5 mm, preferably 0.03 to 2 mm, with particular preference 0.05 to 1 mm.

10. Film-shaped medicament according to any one of the preceding claims, **characterized in that** it contains one or more auxiliaries selected from the group comprising fillers, colourants, emulsifiers, plasticizers, disintegration promoters, disintegrants (wick agents), wetting agents, sweetening and flavouring agents, preservatives, pH regulators, permeation-enhancing substances and antioxidants.

11. Use of at least one cholinergic active agent acting on the central nervous system, selected from the group comprising galanthamine, pharmaceutically acceptable salts of galanthamine, galanthamine derivatives and their pharmaceutically acceptable salts, for the production of a film-shaped buccal medicament intended for transmucosal administration of the said active substance(s), which medicament is soluble in aqueous media or/and disintegrates completely, or essentially completely, within 2 minutes in aqueous media at a temperature of 37°C, but is not mucoadhesive, for treating diseases or disease symptoms accompanied by, or caused by, a lack of acetylcholine-induced conduction and/or a disturbed regulation of neuronal nicotinic receptors.

12. Use according to claim 11, **characterised in that** said film-shaped medicament disintegrates completely, or essentially completely, in aqueous media at a temperature of 37°C within 60 s, preferably within 10 s.

13. Use according to claim 11, **characterized in that** the said disease is Alzheimer's disease or that the said symptom is impaired memory occurring in the course of Alzheimer's disease.

14. Use according to claim 11, **characterized in that** the said treatment is the therapy of alcohol abuse, especially a treatment for reducing the craving for alcohol, or the therapy of nicotine abuse, especially a treatment for reducing the craving for nicotine.

15. Use according to claim 11, **characterized in that** the said treatment is an antidote treatment following neuroleptic anaesthesia.

16. Use according to claim 11, **characterized in that** the said treatment is a therapy of abuse of chemical substances or of the dependence on such substances, especially a therapy of intoxication with psychotropic substances.

17. Use according to claim 11, **characterized in that** the said symptoms or diseases are symptoms of jet lag or other disorders of the physiological rhythm of body functions.

18. Use according to claim 11, **characterized in that** the said symptoms or diseases are chronic fatigue syndrome or disturbed sleep.

19. Use according to claim 11, **characterized in that** the said disease is schizophrenia or a mania.

20. Use according to claim 11, **characterized in that** the said diseases or symptoms are neurological illnesses and symptoms, especially paralytic symptoms.

21. Use according to claim 11, **characterized in that** the medicament is used for the treatment of disorders of the central nervous system occurring as a consequence of the action of psychotropic substances caused by occasional or chronic use or abuse of addictive substances, narcotics or medicaments, or as a side effect of the use of medicaments as intended, especially repeated or prolonged use of medicaments, or as a consequence of acute poisoning, or as a consequence of the chronic action of poisons, in humans or other vertebrates.

22. Use according to claim 21, **characterized in that** the said symptoms are symptoms from the group comprising cognitive disorders, especially impaired memory, as well as impairment of memory performance, impaired perception, impaired coordination of movements.

## Revendications

1. Médicament pelliculaire pour l'administration par voie buccale de galanthamine ou d'un de ses sels ou d'un de ses dérivés, le médicament présentant au moins une couche qui contient une substance active cholinergique agissant sur le système nerveux central, ou bien une combinaison d'au moins deux substances actives de ce type, la/les substances actives étant choisies parmi le groupe comprenant la galanthamine, des sels pharmaceutiquement acceptables de la galanthamine, des dérivés de la galanthamine et leurs sels pharmaceutiquement acceptables, et le médicament pelliculaire étant soluble dans des milieux aqueux et/ou se décomposant complètement ou de manière essentiellement complète dans des milieux aqueux à une température de 37°C dans un laps de temps de 2 minutes, sans toutefois être mucoadhésif.

2. Médicament pelliculaire selon la revendication 1, **caractérisé en ce que** le médicament pelliculaire se décompose complètement ou de manière essentiellement complète dans des milieux aqueux à une température de 37°C dans un laps de temps de 60 secondes, de préférence dans un laps de temps de 10 secondes.

3. Médicament pelliculaire selon la revendication 1 ou 2, **caractérisé en ce que** la couche en question ou au moins une des couches en question présente une matrice polymère qui fait office de réservoir pour la substance active, la fraction polymère s'élevant de 5 à 95 % en poids, de préférence de 15 à 75 % en poids, de manière particulièrement préférée de 20 à 50 % en poids.

4. Médicament pelliculaire selon l'une quelconque des revendications précédentes, **caractérisé en ce qu'**il libère, dans un laps de temps de 30 minutes, de préférence dans un laps de temps de 15 minutes, de manière particulièrement préférée dans un laps de temps de 5 minutes après son application, dans la cavité buccale, une quantité de la/des substances actives qu'il contient telle que l'on obtient un taux plasmatique efficace.

5. Médicament pelliculaire selon l'une quelconque des revendications précédentes, **caractérisé en ce qu'**il possède une structure bicouche ou multicouche, au moins une couche contenant la/les substances actives.

6. Médicament pelliculaire selon l'une quelconque des revendications précédentes, **caractérisé en ce qu'**au moins une des couches présente une libération retardée de la/des substances actives.

7. Médicament pelliculaire selon l'une quelconque des revendications précédentes, **caractérisé en ce que** la teneur en substances actives s'élève de 0,1 à 30 % en poids, de préférence de 1 à 20 % en poids, chaque fois rapportés à la couche/aux couches contenant la/les substances actives.

8. Médicament pelliculaire selon l'une quelconque des revendications précédentes, **caractérisé en ce que** le médicament contient de la galanthamine, ou un sel ou un dérivé de la galanthamine, en combinaison avec au moins une autre substance active pharmaceutique choisie de préférence parmi le groupe des inhibiteurs de l'acétylcholinestérase.

9. Médicament pelliculaire selon l'une quelconque des revendications précédentes, **caractérisé en ce que** son épaisseur de couche s'élève de 0,01 à 5 mm, de préférence de 0,03 à 2 mm, de manière particulièrement préférée de 0,05 à 1 mm.

10. Médicament pelliculaire selon l'une quelconque des revendications précédentes, **caractérisé en ce qu'**il contient une ou plusieurs substances auxiliaires choisies parmi le groupe comprenant des charges, des colorants, des émulsifiants, des plastifiants, des agents favorisant la décomposition, des désintégrants (agents de mèche), des agents mouillants, des édulcorants et des aromatisants, des conservateurs, des régulateurs du pH, des substances favorisant la perméation et des antioxydants.

11. Utilisation d'au moins une substance active cholinergique agissant sur le système nerveux central, choisie parmi le groupe comprenant la galanthamine, des sels pharmaceutiquement acceptables de la galanthamine, des dérivés de la galanthamine et leurs sels pharmaceutiquement acceptables, pour la préparation d'un médicament pelliculaire pour la cavité buccale destiné à une administration par la voie transmuqueuse de la/des substances actives mentionnées, qui est soluble dans des milieux aqueux et/ou qui se décompose complètement ou de manière essentiellement complète dans des milieux aqueux à une température de 37 °C dans un laps de temps de 2 minutes, sans toutefois être mucoadhésif, pour le traitement de maladie ou de symptômes de maladies qui dérivent d'un manque d'influx nerveux transmis par l'acétylcholine et/ou d'un dérèglement des récepteurs nicotiniques neuronaux ou qui sont provoquées par ledit manque et/ou par ledit dérèglement.

12. Utilisation selon la revendication 11, **caractérisée en ce que** le médicament pelliculaire se décompose complètement ou de manière essentiellement complète dans des milieux aqueux à une température de 37 °C dans un laps de temps de 60 secondes, de préférence dans un laps de temps de 10 secondes.

13. Utilisation selon la revendication 11, **caractérisée en ce qu'**il s'agit, en ce qui concerne la maladie mentionnée, de la maladie d'Alzheimer, ou bien **en ce qu'**il s'agit, en ce qui concerne les symptômes mentionnés, des troubles de mémoire qui apparaissent lors de l'évolution de cette maladie.

14. Utilisation selon la revendication 11, **caractérisée en ce qu'**il s'agit, en ce qui concerne le traitement mentionné, de la thérapie de l'abus d'alcool, en particulier d'un traitement pour réduire l'état de manque dû à l'absence d'alcool, ou bien de la thérapie de l'abus de nicotine, en particulier d'un traitement pour la réduction de l'état de manque dû à l'absence de nicotine.

15. Utilisation selon la revendication 11, **caractérisée en ce qu'**il s'agit, en ce qui concerne le traitement mentionné, d'un traitement antidote après une anesthésie aux neuroleptiques.

16. Utilisation selon la revendication 11, **caractérisée en ce qu'**il s'agit, en ce qui concerne le traitement mentionné, d'une thérapie de l'abus de substances chimiques ou de la dépendance de substances de ce type, en particulier d'une thérapie de l'intoxication avec des substances psychotropes.

17. Utilisation selon la revendication 11, **caractérisée en ce qu'**il s'agit, en ce qui concerne les maladies ou les symptômes mentionnés, de symptômes dus au décalage horaire ou d'autres perturbations du rythme physiologique de fonctions corporelles.

18. Utilisation selon la revendication 11, **caractérisée en ce qu'**il s'agit, en ce qui concerne les maladies ou les symptômes mentionnés, du syndrome de fatigue chronique ou de perturbations du sommeil.

19. Utilisation selon la revendication 11, **caractérisée en ce qu'**il s'agit, en ce qui concerne la maladie mentionnée, de la schizophrénie ou d'une manie.

20. Utilisation selon la revendication 11, **caractérisée en ce qu'**il s'agit, en ce qui concerne les maladies ou les symptômes mentionnés, de maladies et de symptômes neurologiques, en particulier de phénomènes de paralysie.

21. Utilisation selon la revendication 11, **caractérisée en ce que** le médicament est utilisé pour le traitement de perturbations du système nerveux central qui apparaissent suite à l'effet de substances psychotropes faisant suite à un usage ou à un abus occasionnel ou chronique de drogues toxicomanogènes, de stupéfiants ou de médicaments, ou comme effet secondaire dans le cas d'une utilisation conforme à une prescription, en particulier dans le cas d'une utilisation répétée ou de longue durée de médicament, ou bien suite à une intoxication aiguë, ou encore suite à l'effet chronique de toxine, chez l'homme ou chez d'autres vertébrés.

22. Utilisation selon la revendication 21, **caractérisée en ce qu'**il s'agit, en ce qui concerne les symptômes mentionnés, de symptômes choisis parmi le groupe comprenant des troubles cognitifs, en particulier des troubles de la mémoire, ainsi qu'une détérioration de la capacité à mémoriser, une détérioration de la perception, une détérioration de la coordination des mouvements.
